# EUROPEAN PATENT APPLICATION

(11) **EP 2 371 292 A2**
(43) Date of publication of application: **05.10.2011**
(21) Application number: 09827700.7
(22) Date of filing: 17.11.2009
(51) Int. Cl.: A61B 10/00

(54) **BIOPSY APPARATUS**

(30) Priority: 18.11.2008 KR 20080114471
(71) Applicant: Park, Hee Boong, Seoul 135-842 (KR)
(72) Inventor: Park, Hee Boong, Seoul 135-842 (KR)
(74) Representative: Beier, Ralph
(86) International application number: PCT/KR2009/006741
(87) International publication number: WO 2010/058935

(57) **Abstract**

A biopsy device for use in excising tissue from a human body includes an outer member, an inner member and a rotary drive means for rotating the inner member with respect to the outer member. The outer member includes a cylindrical outer body portion, an outer cutout portion defined in the outer body portion, an acute outer knife edge portion formed to extend along an edge of the outer cutout portion and a cutting tip provided at a tip end of the outer body portion. The inner member includes an inner body portion inserted into the outer member, an inner cutout portion defined in the inner body portion to obliquely extend with respect to a longitudinal direction of the inner body portion and an acute inner knife edge portion formed to extend along an edge of the inner cutout portion.

## Description

### [Field of the Invention]

The present invention relates to a biopsy device used in removing a piece of tissue from a human body for examination purposes and, more particularly, to a biopsy device having a structure capable of enjoying enhanced tissue removal performance while reducing discomforts felt by a patient.

### [Background Art]

In general, a biopsy device includes a needle type member which can be pricked into a target tissue area to remove a piece of tissue. This biopsy device is used to remove soft tissue other than bones. In recent years, it is often the case that the tissue of a breast is removed and examined with a biopsy device. The breast tissue is soft in some areas but very tough and hard in other areas, particularly in fibrous tissue areas. It is therefore important that the biopsy device for removal and examination of breast tissue have superior excision performance so that it can effectively excise tough breast tissue.

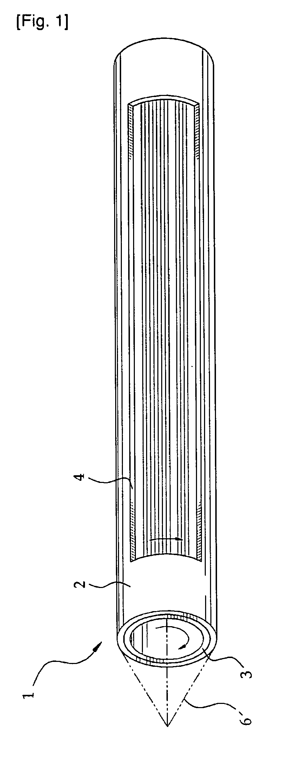

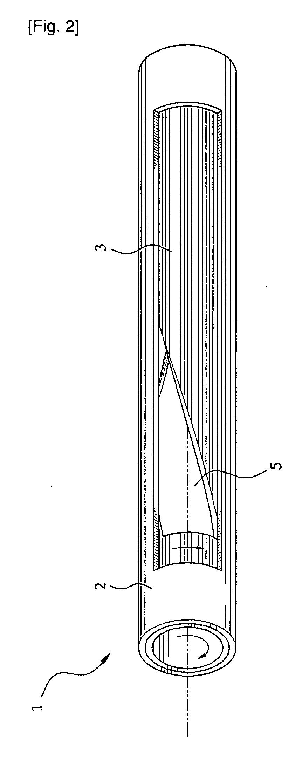

Figs. 1 and 2 shows a biopsy device 1 disclosed in International Publication W02004/075719. The biopsy device 1 includes an outer member 2 and an inner member 3, both of which have a cylindrical shape. The inner member 3 is inserted into the outer member 2. The outer member 2 and the inner member 3 are respectively provided with excising portions 4 and 5. The outer member 2 includes a cutting tip 6 that can incise tissue and move inwards. As shown in Fig. 2, the tissue of a human body is partially put between the excising portions 4 and 5. The tissue is excised as the inner member 3 makes rotation with respect to the outer member 2.

The biopsy device 1 of this structure suffers from a problem of reduced tissue excision performance. In particular, it is quite difficult for the biopsy device 1 to effectively excise tough tissue such as fibrous breast tissue. Only the excising portion 5 of the inner member 3 has a knife edge. The excising portion 4 of the outer member 2 is provided with no knife edge. This makes it difficult to effectively excise tissue.

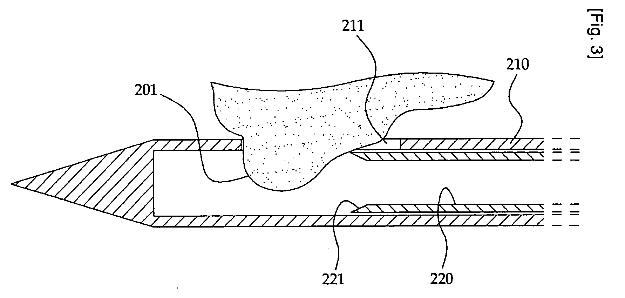

Fig. 3 is a section view showing another example of conventional biopsy devices. This biopsy device includes an outer member 210 and an inner member 220 which can move back and forth while making rotation with respect to the outer member 210. The outer member 210 has an excising portion 211. The inner member 220 is provided at its tip end with a terminal knife edge portion 221. If the inner member 220 moves forward while making rotation, the tissue placed within the excising portion 211 of the outer member 210 is cut by the terminal knife edge portion 221.

In case of the conventional biopsy device having such a structure, if the playing gap between the inner member 220 and the outer member 210 is small, the inner member 220 cannot be rotated at a high speed due to the increased friction between the inner member 220 and the outer member 210. Therefore, the reduction in the playing gap between the inner member 220 and the outer member 210 results in a decrease in the rotation speed of the inner member 220, thereby making it impossible to effectively excise tough tissue.

On the other hand, if the playing gap between the inner member 220 and the outer member 210 is set greater, it becomes possible to reduce friction between the inner member 220 and the outer member 210 and to rotate the inner member 220 at a high speed. However, the increase in the playing gap between the inner member 220 and the outer member 210 makes it difficult to cut the tissue 201.

In case where the inner member 220 is rotated at a specified speed or more, it is possible to effectively excise tissue. However, difficulties are encountered in increasing the rotation speed of the inner member 220 to the specified speed. If the rotation speed of the inner member 220 is decreased due to the load applied to the inner member 220 by the resistance of tough tissue during the excision process, the tissue excision performance of the biopsy device gets lowered. If the inner member 220 is rotated at a high speed in an effort to enhance the tissue excision performance, a great deal of vibration is generated. This poses a problem in that a patient feels uneasy and anxious.

### [Detailed Description of the Invention]

### [Technical Problems]

In view of the problems mentioned above, it is an object of the present invention to provide a biopsy device capable of efficiently excising the tissue of a human body for examination purposes.

Another object of the present invention is to provide a biopsy device having a structure suitable for reducing vibration.

### [Solution to the Technical Problems]

In one aspect of the present invention, there is provided a biopsy device for use in excising tissue from a human body, including:
an outer member including a cylindrical outer body portion, an outer cutout portion defined in the outer body portion to permit entry of the tissue into the outer body portion therethrough, an acute outer knife edge portion formed to extend along an edge of the outer cutout portion and a cutting tip provided at a tip end of the outer body portion to incise the tissue when the outer body portion is driven into a human body;
an inner member including an inner body portion inserted into the outer member, an inner cutout portion defined in the inner body portion to obliquely extend with respect to a longitudinal direction of the inner body portion and an acute inner knife edge portion formed to extend along an edge of the inner cutout portion; and
a rotary drive means for rotating the inner member with respect to the outer member such that the inner knife edge portion moves across the outer knife edge portion to cut the tissue placed between the inner knife edge portion and the outer knife edge portion.

### [Advantageous Effects]

With the biopsy device according to the present invention, it is possible to efficiently excise a piece of tissue through the use of an outer knife edge portion and an inner knife edge portion. This assists in simplifying the biopsy operation and shortening the operation time.

Moreover, the biopsy device according to the present invention can be operated with reduced vibration. This helps reduce uneasiness and anxiety felt by a patient during a biopsy operation.

### [Brief Description of the Drawings]

Fig. 1 is a perspective view showing major parts of a conventional biopsy device.
Fig. 2 is a perspective view for explaining how to operate the biopsy device shown in Fig. 1.
Fig. 3 is a section view showing another conventional biopsy device.
Fig. 4 is a section view showing a biopsy device according to one embodiment of the present invention.
Fig. 5 is a partially enlarged perspective view of the biopsy device shown in Fig. 4.
Fig. 6 is a perspective view showing an inner member employed in the biopsy device shown in Fig. 4.
Fig. 7 is a section view taken along line VI-VI in Fig. 5.
Figs. 8 through 10 are views for explaining how to operate the biopsy device shown in Fig. 4.
Fig. 11 is a perspective view showing an inner member employed in a biopsy device according to another embodiment of the present invention.
Fig. 12 is a perspective view showing a biopsy device according to a further embodiment of the present invention.
Fig. 13 is a perspective view showing an inner member employed in the biopsy device shown in Fig. 12.

### [Best Mode for Carrying out the Invention]

Hereinafter, certain preferred embodiments of a biopsy device according to the present invention will be described with reference to the accompanying drawings.

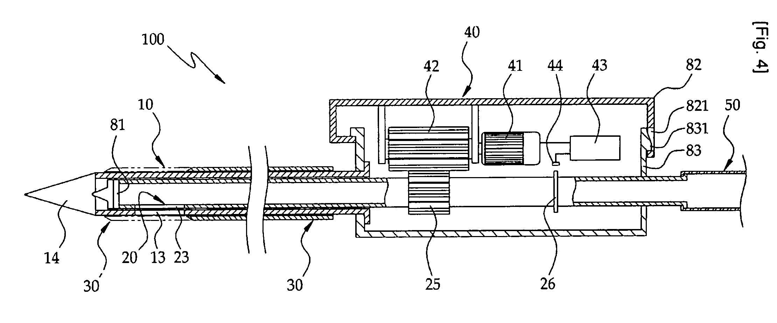

Referring to Figs. 4 through 6, a biopsy device 100 according to one embodiment of the present invention includes an outer member 10, an inner member 20, a rotary drive unit 40 and a cover member 30.

The outer member 10 includes a cylindrical outer body portion 11 having an outer cutout portion 12 through which the inside and outside of the outer member 10 can communicate with each other. When the outer body portion 11 is driven into a human body, the tissue making contact with the outer surface of the outer body portion 11 comes into outer body portion 11 through the outer cutout portion 12.

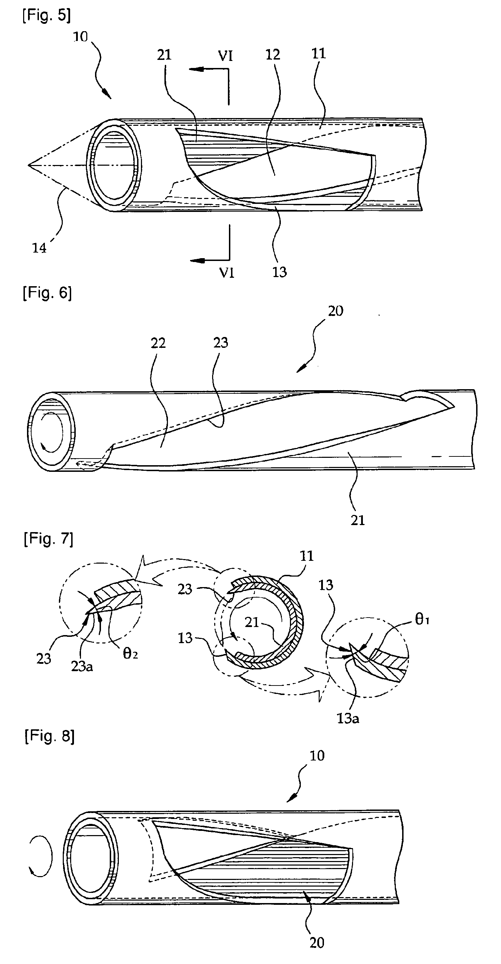

The outer cutout portion 12 is provided at one edge thereof with an outer knife edge portion 13 having an acute knife edge surface 13a. As can be seen in Fig. 7, the inner surface of the outer body portion 11 makes an acute angle θ1 with the knife edge surface 13a.

The outer body portion 11 of the outer member 10 is provided at a tip end thereof with an acute cutting tip 14. When the outer member 10 is driven into a human body by a pushing force, the cutting tip 14 incises the skin and tissue of the human body so that the outer member 10 can cut into the human body with ease.

The inner member 20 includes a cylindrical inner body portion 21 inserted into the outer member 10. The inner body portion 21 has an inner cutout portion 22 formed by partially removing the inner body portion 21. The inner cutout portion 22 is formed to extend obliquely with respect to the longitudinal direction of the inner body portion 21.

The inner cutout portion 22 is provided at one edge thereof with an inner knife edge portion 23 having an acute knife edge surface 23a. As can be seen in Fig. 7, the outer surface of the inner body portion 21 makes an acute angle θ2 with the knife edge surface 23a. The inner knife edge portion 23 is formed to helically extend along the outer surface of the inner body portion 21.

As shown in Fig. 4, the inner member 20 is inserted into the outer member 10, preferably in an axially compressed state. More specifically, an elastically-deformable biasing member 81 is first arranged near the tip end of the inner member 20. Then, the inner member 20 is pushed into the outer member 10 by applying a force to the inner member 20. The opposite extension of the inner member 20 is rotatably supported on a housing to be set forth later. In this manner, the inner member 20 can be installed in an axially compressed state. Thus, the inner member 20 is elastically deformed so that it can be axially compressed but radially expanded by the axial compressing force. Radial expansion of the inner member 20 ensures that the inner knife edge portion 23 and the outer knife edge portion 13 are kept in close contact with each other.

The outer knife edge portion 13 is formed to obliquely extend along the outer body portion 11. The outer knife edge portion 13 is inclined in a direction opposite to the extension direction of the inner knife edge portion 23.

Referring again to Fig. 4, the cover member 30 is formed into a cylindrical shape and is arranged to partially cover the outer member 10. The cover member 30 is axially slidable with respect to the outer member 10.

The rotary drive unit 40 is operatively connected to the inner member 20 so that it can rotate the inner member 20 with respect to the outer member 10. The rotary drive unit 40 is configured to rotate the inner member 20 with respect to the outer member 10 in such a direction that the inner knife edge portion 23 and the outer knife edge portion 13 meet in a mutually intersecting relationship to cut the tissue of a human body placed therebetween. In other words, the rotary drive unit 40 is designed to rotate the inner member 20 with respect to the outer member 10 so that the inner knife edge portion 23 can intersect the outer knife edge portion 13 at continuously varying points.

The rotary drive unit 40 includes a motor 41 and a driving gear 42 directly connected to the motor 41. The driving gear 42 meshes with a driven gear 25 provided on the outer surface of the inner body portion 21 of the inner member 20. The motor 41 may preferably be a servo motor. The motor 41 is electrically connected to a control unit 43 for controlling the rotation speed and angular displacement of the motor 41. The control unit 43 controls the angular displacement of the motor 41 based on the detection results of angular displacement sensor members 26 and 44 respectively connected to inner member 20 and the control unit 43. By controlling the relative position between the inner cutout portion 22 and the outer cutout portion 12, the control unit 43 enables the inner member 20 to open or close the outer cutout portion 12. At this time, an operator can grasp the relative position between the inner cutout portion 22 and the outer cutout portion 12.

Preferably, the outer knife edge portion 13 is formed to obliquely extend along the outer body portion 11 such that the outer knife edge portion 13 is inclined in a direction opposite to the extension direction of the inner knife edge portion 23. It is also preferred that the frontal end extension of the outer knife edge portion 13 be formed into a curved shape as shown in Fig. 5.

A vacuum suction unit 50 for drawing and discharging an excised piece of tissue is connected to the inner member 20. A filtering net (not shown) may be provided in the vacuum suction unit 50 to collect the excised piece of tissue while allowing liquid components such as blood to pass therethrough.

A water supply nozzle may be provided at the tip end of the outer member 10 to supply water to the tissue through the water supply nozzle. The water thus supplied helps the excised tissue and blood to move toward the vacuum suction unit 50. In this case, the control unit 43 controls not only the operation of the vacuum suction unit 50 but also the supply of water to the water supply nozzle.

As can be seen in Fig. 4, the motor 41, the driving gear 42 and the control unit 43 are arranged within a first housing 82. The outer member 10 and the inner member 20 are arranged within a second housing 83. The second housing 83 has an engagement hook 831 that engages with an engagement hole 821 formed in the first housing 82. Thus, the first housing 82 and the second housing 83 are coupled together in a removable manner. This makes it possible to easily replace those parts driven into a human body, such as the outer member 10 and the inner member 20.

Next, description will be made on the operation of the present biopsy device configured as above.

Upon pressing the cutting tip 14 of the biopsy device 100 against the skin of a human body, the cutting tip 14 incises the skin and moves into the tissue to be excised. Then, the cover member 30 is caused to move forward along the outer member 10 to close the outer cutout portion 12, in which state the outer member 10 is driven into the human body. At this time, the tissue is not caught by the outer cutout portion 12 because the cover member 30 keeps the outer cutout portion 12 closed.

Subsequently, the cover member 30 is caused to slide backward along the outer member 10 so that the outer cutout portion 12 can be opened. The vacuum suction unit 50 is operated to reduce the internal pressure of the inner member 20. As a result, the tissue is drawn into the inner member 20 through the outer cutout portion 12 and the inner cutout portion 22.

As shown in Figs. 8 through 10, the inner member 20 is rotated by operating the rotary drive unit 40 in a state that the outer cutout portion 12 and the inner cutout portion 22 overlap with each other over a relatively wide area. The inner member 20 rotates with respect to the outer member 10 in a direction indicated by an arrow. Consequently, the inner knife edge portion 23 moves across the outer knife edge portion 13 to cut a piece of tissue.

Referring to Fig. 6, the inner knife edge portion 23 is formed to helically extend counterclockwise toward the tip end of the inner member 20. The rotary drive unit 40 rotates the inner member 20 in a direction in which the front end of the inner knife edge portion 23 leads the rear end thereof, namely in the counterclockwise direction when seen from the right end in Fig. 6. As the inner member 20 is rotated in this direction, the inner knife edge portion 23 moves across the outer knife edge portion 13, thereby cutting a piece of tissue in an efficient manner.

If the inner member 20 is twisted in the same direction as the helical extension direction of the inner knife edge portion 23, the inner cutout portion 22 grows narrower to reduce the outer diameter of the inner member 20. In contrast, if the inner member 20 is twisted in the opposite direction to the helical extension direction of the inner knife edge portion 23, the inner cutout portion 22 grows wider to increase the outer diameter of the inner member 20. In the present embodiment, the tissue cutting performance can be enhanced using the principle that the outer diameter of the inner member 20 is increased upon twisting the inner member 20 in a specified direction.

If the inner member 20 is rotated in a direction in which the front end of the inner knife edge portion 23 leads the rear end thereof, the inner knife edge portion 23 is expanded outwards by the resistance of the tissue placed within the inner member 20. Thus, the inner knife edge portion 23 makes closer contact with the inner surface of the outer member 10. This allows the inner knife edge portion 23 to move across the outer knife edge portion 13 in a closely contacted state.

During rotation of the inner member 20, the inner knife edge portion 23 presses the tissue against the outer knife edge portion 13, not perpendicularly but obliquely, so that the tissue can be gradually cut with an increased shear force by the scissors-like cutting action of the inner knife edge portion 23 and the outer knife edge portion 13. At this time, the inner knife edge portion 23 intersects the outer knife edge portion 13 at continuously varying intersection points. Under this principle, it is possible to cut a piece of tissue in an easy and efficient manner.

As mentioned earlier with reference to Fig. 4, the inner member 20 is axially compressed by the biasing member 81. Thus, the inner member 20 is elastically deformed, i.e., radially expanded, to have a reduced length and an increased diameter. This assists in bringing the inner knife edge portion 23 and the outer knife edge portion 13 into close contact with each other, which helps enhance the tissue cutting performance of the biopsy device.

As can be seen in Fig. 7, the inner surface of the outer body portion 11 makes an acute angle θ1 with the knife edge surface 13a of the outer knife edge portion 13. The outer surface of the inner body portion 21 makes an acute angle θ2 with the knife edge surface 23a of the inner knife edge portion 23. This enables the inner knife edge portion 23 and the outer knife edge portion 13 to cut the tissue in a reliable manner.

As described above, the present biopsy device 100 is designed to excise a piece of tissue using the scissors-like cutting action of the inner knife edge portion 23 and the outer knife edge portion 13. Thanks to this feature, the tissue cutting performance of the present biopsy device 100 is superior to that of the conventional biopsy device shown in Fig. 3. This makes it possible to easily cut tissue without having to rotate the inner member 20 at an increased speed, which helps reduce the uneasiness and anxiety felt by a patient. The reduced rotation speed of the inner member 20 enables then operator to precisely and accurately perform the tissue excision operation.

Using the control unit 43 and the motor 41, the operator can grasp the position (angular displacement) of the inner cutout portion 22 with respect to the outer cutout portion 12 when performing the tissue excision operation. This enables the operator to accurately and efficiently excise a piece of tissue while confirming the position of the tissue to be excised. This reduces or eliminates the possibility that other tissue than the target tissue is excised by mistake.

The piece of tissue excised is discharged to the outside by operating the vacuum suction unit 50. If water is supplied to the tip end of the outer member 10, it becomes possible to discharge the piece of tissue in an efficient manner. Moreover, if a filtering net is provided in the vacuum suction unit 50, it is possible to collect the excised piece of tissue while allowing liquid components such as blood to pass therethrough.

At the end of the tissue excision operation, the cover member 30 is caused to move forward along the outer member 10 so that the outer cutout portion 12 of the outer member 10 can be covered by the cover member 30. If the biopsy device 100 is pulled away from the human body in this state, the outer member 10 can be easily removed from the tissue with no possibility that the tissue is caught by the outer cutout portion 12.

While one preferred embodiment processing furnace 202 the present invention has been described above, the present invention is not limited to this embodiment.

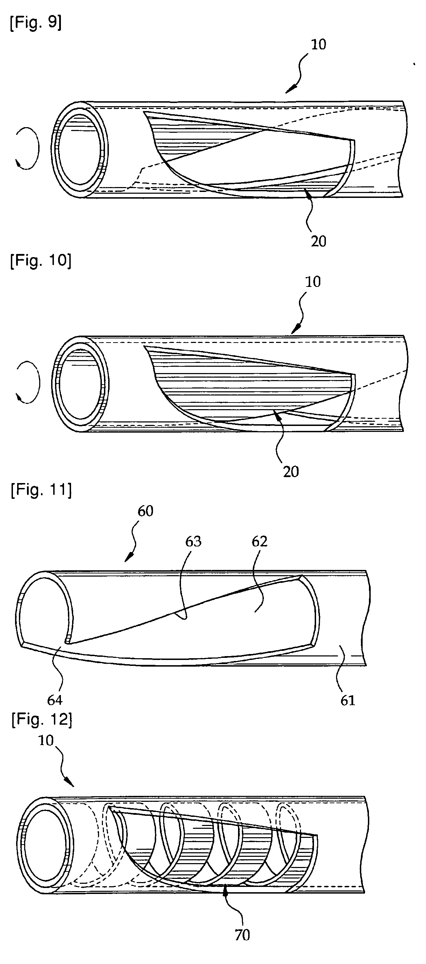

As one modified embodiment, there may be provided a biopsy device including an inner member 60 as shown in Fig. 11. Just like the inner member 20 described earlier, the inner member 60 shown in Fig. 11 includes an inner body portion 61, an inner cutout portion 62 and an inner knife edge portion 63. In the inner member 60, however, the inner cutout portion 62 is not closed but opened at the tip end of the inner member 60. In other words, the inner member 60 includes an end opening portion 64 formed in the tip end portion of the inner body portion 61 so that the inner cutout portion 62 can be opened toward the tip end of the inner member 60 through the end opening portion 64.

The inner member 20 may be formed to have an outer diameter a little greater than the inner diameter of the outer member 10. When inserting the inner member 20 into the outer member 10, the inner member 20 is elastically deformed so that the outer diameter thereof can become smaller than the inner diameter of the outer member 10. After insertion of the inner member 20 into the outer member 10, the inner member 20 tends to recover its original shape and therefore makes close contact with the outer member 10. Formation of the end opening portion 64 in the inner body portion 61 makes it easy to elastically deform the inner member 60 to have a reduced diameter.

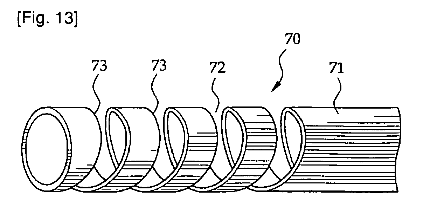

As another modified embodiment, there may be provided a biopsy device including an inner member 70 as shown in Figs. 12 and 13. Just like the inner member 20 described earlier, the inner member 70 shown in Fig. 13 includes an inner cutout portion 72 and an inner knife edge portion 73, both of which are formed to helically extend along the outer surface of the inner member 20 over an extent of 360° or more. The inner member 70 shown in Fig. 13 differs from the inner member 20 shown in Fig. 6 in that the inner cutout portion 72 and the inner knife edge portion 73 extend over an extent of 360° or more.

### [Industrial Applicability]

The biopsy device according to the present invention can be used in excising the tissue of a human body for examination purposes.

## Claims

1. A biopsy device for use in excising tissue from a human body, comprising:
an outer member including a cylindrical outer body portion, an outer cutout portion defined in the outer body portion to permit entry of the tissue into the outer body portion therethrough, an acute outer knife edge portion formed to extend along an edge of the outer cutout portion and a cutting tip provided at a tip end of the outer body portion to incise the tissue when the outer body portion is driven into a human body;
an inner member including an inner body portion inserted into the outer member, an inner cutout portion defined in the inner body portion to obliquely extend with respect to a longitudinal direction of the inner body portion and an acute inner knife edge portion formed to extend along an edge of the inner cutout portion; and
a rotary drive means for rotating the inner member with respect to the outer member such that the inner knife edge portion moves across the outer knife edge portion to cut the tissue placed between the inner knife edge portion and the outer knife edge portion.

2. The biopsy device as recited in claim 1, wherein the outer knife edge portion has a knife edge surface making an acute angle with an inner surface of the outer body portion, and the inner knife edge portion has a knife edge surface making an acute angle with an outer surface of the inner body portion.

3. The biopsy device as recited in claim 2, wherein the outer knife edge portion is formed to obliquely extend with respect to a longitudinal direction of the outer body portion in a direction opposite to an extension direction of the inner knife edge portion.

4. The biopsy device as recited in claim 2, wherein the inner knife edge portion is formed to helically extend along an outer surface of the inner body portion.

5. The biopsy device as recited in any one of claims 1 to 3, wherein the inner member is elastically deformable such that, when inserted into the outer member, the inner member 20 is elastically expanded into close contact with an inner surface of the outer member.

6. The biopsy device as recited in claim 5, wherein the inner member includes an end opening portion formed in a tip end portion of the inner body portion to open the inner cutout portion toward a tip end of the inner member.

7. The biopsy device as recited in claim 5, wherein the inner cutout portion and the inner knife edge portion are formed to helically extend along an outer surface of the inner body portion over an extend of 360° or more.

8. The biopsy device as recited in any one of claims 1 to 3, wherein the inner member is inserted into the outer member in an axially compressed state.

9. The biopsy device as recited in claim 8, wherein the inner member includes an end opening portion formed in a tip end portion of the inner body portion to open the inner cutout portion toward a tip end of the inner member.

10. The biopsy device as recited in claim 8, wherein the inner cutout portion and the inner knife edge portion are formed to helically extend along an outer surface of the inner body portion over an extend of 360° or more.

11. The biopsy device as recited in claim 1, further comprising: a vacuum suction unit connected to the inner member to discharge an excised piece of tissue from the inner member.

12. The biopsy device as recited in claim 1, wherein the rotary drive means includes a motor for rotating the inner member and a control unit for controlling a rotation speed and an angular displacement of the motor.

13. The biopsy device as recited in claim 1, further comprising: a cover member axially slidably arranged on an outer surface of the outer member.
